Europäisches Patentamt

European Patent Office

Office européen des brevets

(19)

(11) Numéro de publication: **0 169 115**
A1

# (12) DEMANDE DE BREVET EUROPEEN

(21) Numéro de dépôt: 85401209.3

(22) Date de dépôt: 18.06.85

(51) Int. Cl.⁴: **C 07 K 3/10,** C 07 K 15/06, A 61 K 39/39, A 61 K 45/05, A 61 K 37/18

(30) Priorité: 19.06.84 FR 8409560

(43) Date de publication de la demande: 22.01.86
Bulletin 86/4

(84) Etats contractants désignés: **AT BE CH DE FR GB IT LI LU NL SE**

(71) Demandeur: **RHONE-POULENC SANTE, Les Miroirs 18 Avenue d'Alsace, F-92400 Courbevoie Cedex (FR)**

(72) Inventeur: **Jolles, Pierre, 2, rue Jean-François GERBILLON, F-75270 Paris Cedex 06 (FR)**
Inventeur: **Migliore-Samour, Danièle, 64/66, avenue Charles Gide, F-94270 Le Kremlin-Bicetre (FR)**
Inventeur: **Parker, Fabienne, 32, avenue Paul Painlevé, F-94200 St. Maur-Des-Fosses (FR)**

(74) Mandataire: **Pilard, Jacques et al, RHONE-POULENC RECHERCHES Service Brevets Pharma 25, Quai Paul Doumer, F-92408 Courbevoie Cedex (FR)**

(54) Nouvelles substances biologiquement actives, leur procédé de préparation à partir du fibrinogène bovin et les compositions qui les contiennent.

(57) Nouvelles substances biologiquement actives obtenues après traitement du fibrinogène bovin réduit et alkylé par une enzyme protéolytique.

Ces nouvelles substances ont des propriétés immunologiques.

EP 0 169 115 A1

ACTORUM AG

La présente invention concerne des substances biologiquement actives obtenues par fractionnement d'hydrolysats enzymatiques du fibrinogène bovin et les compositions qui les contiennent.

Les substances selon la présente invention sont des agents immunologiques qui favorisent en particulier la production d'anticorps.

Selon la présente invention, les nouvelles substances sont obtenues par traitement, à l'aide d'au moins une enzyme protéolytique, du fibrinogène bovin préalablement réduit par le mercaptoéthanol et alkylé par l'iodoacétamide, suivi du fractionnement des produits hydrosolubles en fonction de leur poids moléculaire moyen par filtration et purification sur des supports appropriés.

Les nouvelles substances selon l'invention qui sont éluées au niveau des substances de poids moléculaire moyen compris entre 500 et 7500 présentent des propriétés particulièrement intéressantes.

A titre d'enzymes protéolytiques, on utilise avantageusement dans le procédé selon l'invention la trypsine, la chymotrypsine ou autre enzyme analogue ou leurs mélanges.

Par exemple, le fractionnement selon le poids moléculaire de la fraction hydrosoluble provenant du fibrinogène bovin traité par le mercaptoéthanol et l'iodoacétamide et digéré par la trypsine non prétraitée fournit trois fractions biologiquement actives IV (MJH 104), V (MJH 105) et VI (MJH 106).

La fraction "V" (MJH 105), après purification sur une colonne d'échangeur d'ions fournit 3 fractions actives /pic 5 (MJH 176, 197, 200); pic 10 (MJH 179); pic 11 (MJH 180)/ puis par chromatographie liquide à haute performance de la fraction "pic 5", on obtient deux zones, 4 (MJH 228) et 5 (MJH 229), contenant des produits actifs. L'une d'entre-elles (zone 5) fournit les substances appelées "MJH 335" et "MJH 336" dont les propriétés immunologiques sont améliorées par rapport à celles de la fraction"V" elle-même.

Les nouvelles substances selon la présente invention sont des agents immunologiques qui favorisent la production d'anticorps et qui accèlèrent le phénomène de phagocytose.

In vitro, ils se sont montrés particulièrement actifs à des concentrations comprises entre 0,1 et 10 µg/ml dans le test de sécrétion d'anticorps (hémolytiques) anti-globules rouges de moutons par des cellules spléniques de souris immunisées in vivo et dans le test de phagocytose de globules rouges de moutons opsonisés par les marcrophages péritonéaux de souris.

Les exemples suivants, donnés à titre non limitatif, montrent comment l'invention peut être mise en pratique.

EXEMPLE

Réduction du fibrinogène bovin et alkylation

500 mg de fibrinogène sont dissous dans 25cm$^3$ de tampon tris-(hydroxyméthyl)-aminométhane 0,1M ajusté au pH 8 par HCl 1M, en présence d'urée 8M. La concentration finale de la protéine est de 20 mg/cm$^3$, le mercaptoéthanol (0,2 cm$^3$) est ajouté à la solution protéique suivant le rapport molaire entre l'agent réducteur et la protéine de 2000 pour 1. La réduction est effectuée à 37°C pendant 3 heures sous atmosphère d'azote. Les résidus de cystéine libérés sont stabilisés par alkylation avec 410 mg d'iodoacétamide, en maintenant le pH du milieu à 8, par addition de quelques gouttes de NaOH 1M.

L'addition de 10 volumes du mélange acétone : acide chlorhydrique (39/1 en volumes) précipite la protéine après 12 heures à 4°C. Une centrifugation à 15000 tours/mn pendant 30 mn permet de séparer le précipité. Il est lavé trois fois par le mélange décrit ci-dessus et trois fois par l'éther. Le précipité est remis en suspension dans l'eau, dialysé et concentré sur une membrane Diaflo UM 10 (Amicon), le produit dialysé est lyophilisé.

Le fibrinogène réduit et alkylé est solubilisé par traitement au moyen d'une solution d'hydroxyde de sodium 0,1M de telle manière que la concentration finale en fibrinogène soit voisine de 1 mg/cm³.

La solution obtenue est soumise à une dialyse contre 2 litres de tampon phosphate 0,033 M à pH 8 pendant 2 jours en renouvelant 5 fois la solution tampon.

On obtient ainsi une solution à pH 8 contenant le fibrinogène soluble. Cette solution est soumise à l'action de la trypsine non prétraitée de telle manière que le rapport enzyme/substrat soit voisin de 1/100. L'hydrolyse enzymatique est poursuivie pendant 24 heures à 37°C, la moitié de la quantité d'enzyme étant introduite au départ de la réaction et le reste 4 heures après le début de l'hydrolyse.

Le mélange réactionnel est centrifugé à 13000 tours/mn pendant 1 heure; le surnageant est amené à sec puis repris par 30 cm³ d'une solution d'acide acétique à 30%. Le mélange est soumis à une centrifugation à 13000 tours/mn, pendant 30 minutes.

Le liquide clair surnageant est filtré sur une colonne de Sephadex G 50 (hauteur : 123 cm, diamètre : 4,5 cm) en éluant avec de l'acide acétique à 30% et en recueillant des fractions de 2,7 cm³.

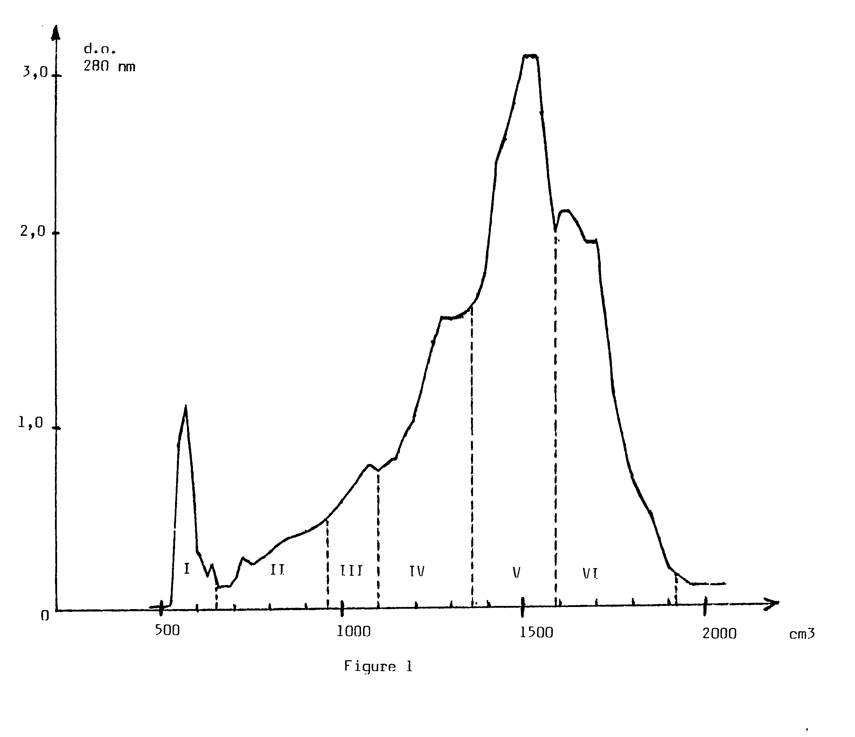

En opérant de cette manière et en suivant la chromatographie par absorption U.V. à 280 nm, on obtient des zones qui permettent de définir des fractions pour lesquelles le poids moléculaire moyen est déterminé. Le diagramme de filtration est représenté par la figure 1.

Les fractions biologiquement actives sont les suivantes :
- substance IV : fractions 1105 à 1369 cm³; poids moléculaire moyen 7000
- substance V : fractions 1371 à 1593 cm³; poids moléculaire moyen 2500

— substance VI : fractions 1594 à 1922 cm$^3$; poids moléculaire moyen 800.

La substance V est filtrée sur une colonne de CM Trisacryl (marque déposée IBF) (hauteur : 110 cm; diamètre 2,2 cm) en éluant avec une solution de tampon tris, HCl $/$tris (hydroxyméthyl aminométhane), HCl$/$ 0,01 M à pH 3,5 et en recueillant des fractions de 3,1 cm$^3$.

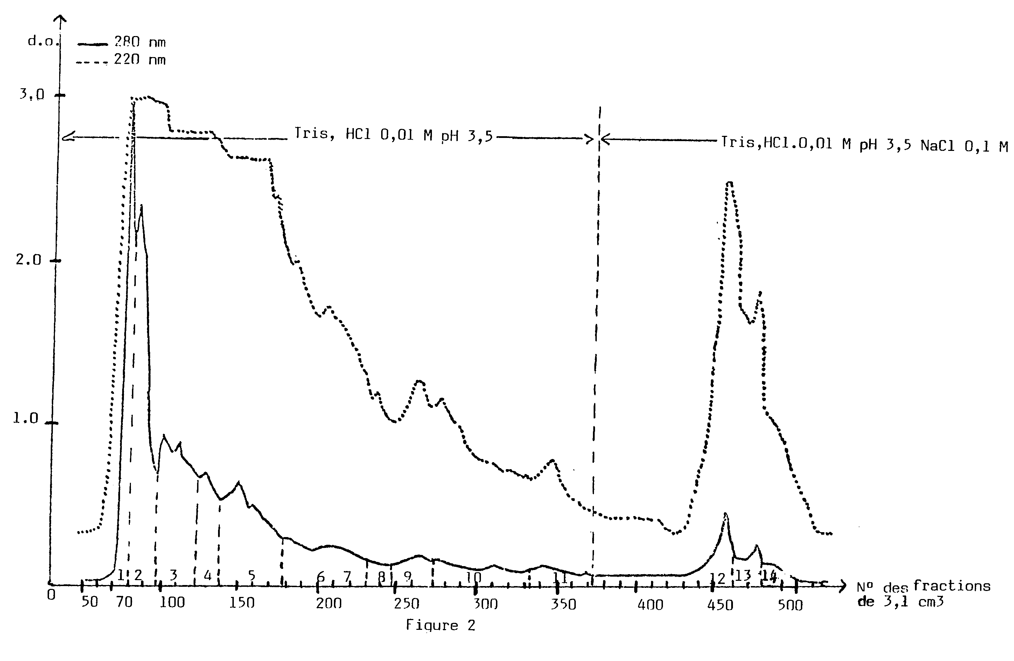

Le diagramme de filtration est représenté par la figure 2 dans laquelle figurent en abscisses les numéros des fractions et en ordonnées les densités optiques à 280 et 220 nm.

On recueille 14 fractions parmi lesquelles les fractions de 430 à 558 cm$^3$, de 850 à 1042 cm$^3$ et de 1043 à 1153 cm$^3$ fournissent respectivement les substances actives appelées "pic 5" (MJH 176, 197 et 250), "pic 10" (MJH 179) et "pic 11" (MJH 180).

La fraction "pic 5" est purifiée par HPLC en phase "reverse" sur une colonne semi-préparative (colonne C18-μ-bondapack WATERS) dont la longueur est de 30 cm et le diamètre de 7,8 mm. On recueille des fractions de 0,5 cm$^3$ la vitesse d'élution étant de 1 cm$^3$/minute. Au départ la colonne est tamponnée par de l'acide trifluoroacétique (TFA) à 0,1% (éluant A).

On prépare un éluant contenant du TFA (0,1% en volume) et de l'acétonitrile (70%) (éluant B).

La fraction "pic 5" est dissoute dans 500 μl de TFA 0,1%.

L'élution est effectuée en utilisant un gradient linéaire d'élution et en opérant selon le tableau suivant :

| Temps (minutes) | Eluant A | Eluant B |
|---|---|---|
| 0 | 100 | 0 |
| 60 | 0 | 100 |

5

0169115

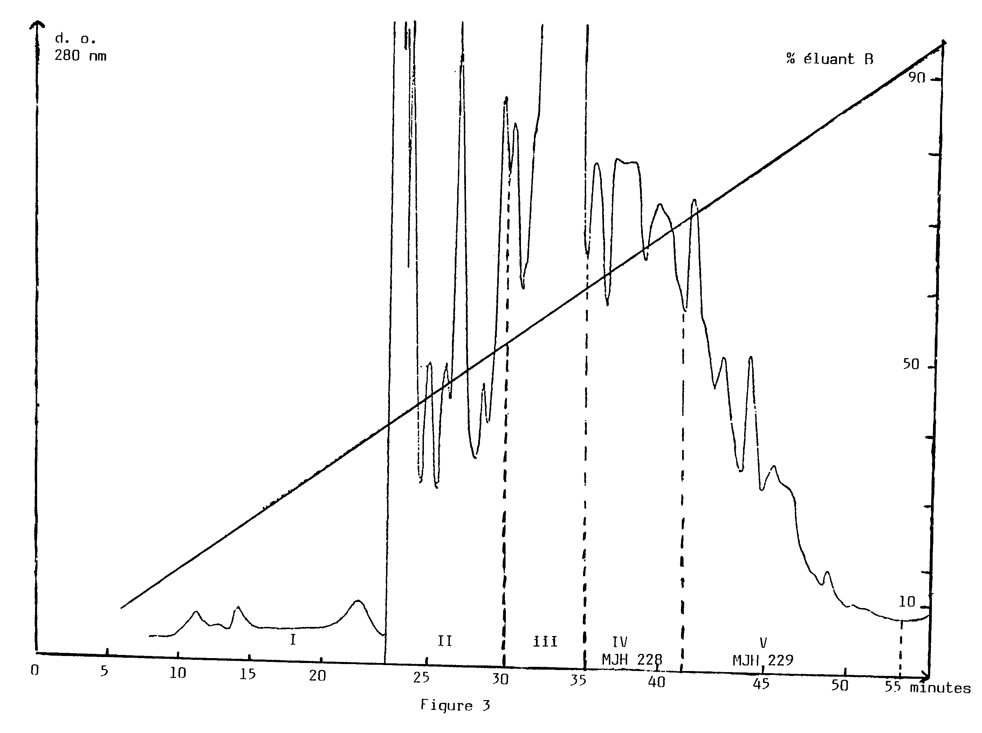

Le diagramme d'élution est représenté par la figure 3. L'élution est suivie en mesurant l'absorption à 280 nm et à 220 nm.

Le diagramme d'élution contient 5 zones dont les fractions les plus importantes sont la zone IV (MJH 228) et la zone V (MJH-229) issues du mélange d'élution pour un temps de rétention compris respectivement entre 36 et 41 minutes et 41 et 55 minutes.

La fraction MJH-228 est à nouveau purifiée par HPLC sur une colonne de même nature que celle utilisée précédemment, en recueillant des fractions de 0,5 cm$^3$, la vitesse d'élution étant de 1 cm$^3$/minute.

On utilise les mêmes éluants que précédemment.

L'élution est effectuée en utilisant un gradient hyperbolique n° 7 (WATERS) d'élution et en opérant selon le tableau suivant :

| Temps (minutes) | Eluant A | Eluant B |
| --- | --- | --- |
| 0 | 100 | 0 |
| 10 | 90 | 10 |
| 70 | 0 | 100 |

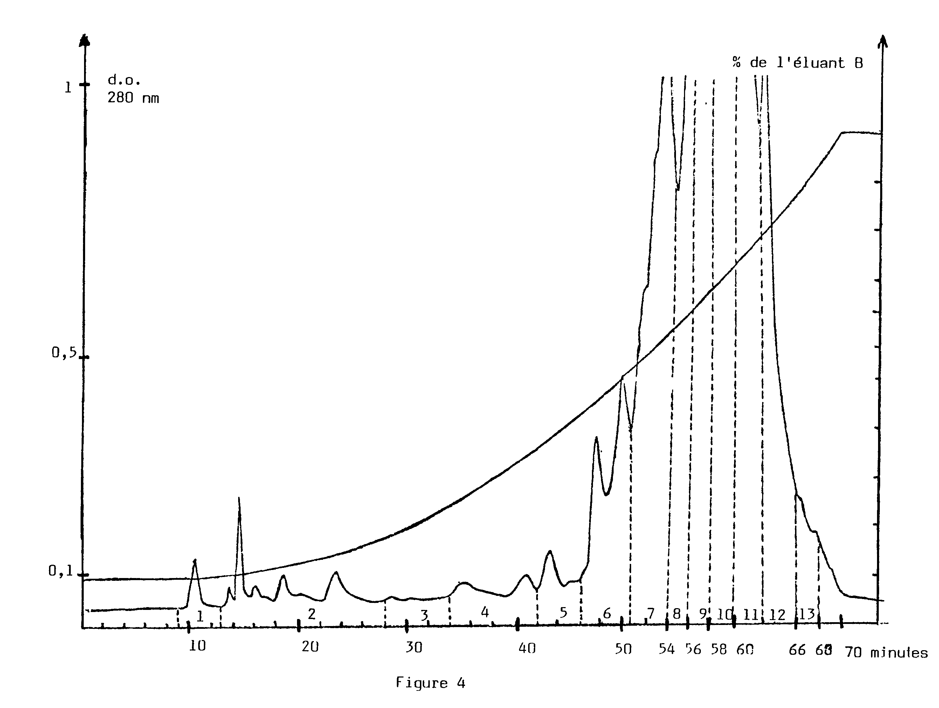

Le diagramme d'élution est représenté par la figure 4.

Le diagramme d'élution contient 13 pics.

Les fractions éluées entre 51 et 54 (pic 7), 54 et 56 (pic 8), 61 et 63 (pic 11) et 63 et 66 minutes (pic 12) contiennent respectivement les substances actives MJH-257, MJH -258, MJH-261 et MJH 262.

La fraction MJH-229 est à nouveau purifiée par HPLC sur une colonne de même nature que celle utilisée précédemment en recueillant des fractions de 0,5 cm$^3$, la vitesse d'élution étant de 1 cm$^3$/minute.

On utilise les mêmes éluants que précédemment.

0169115

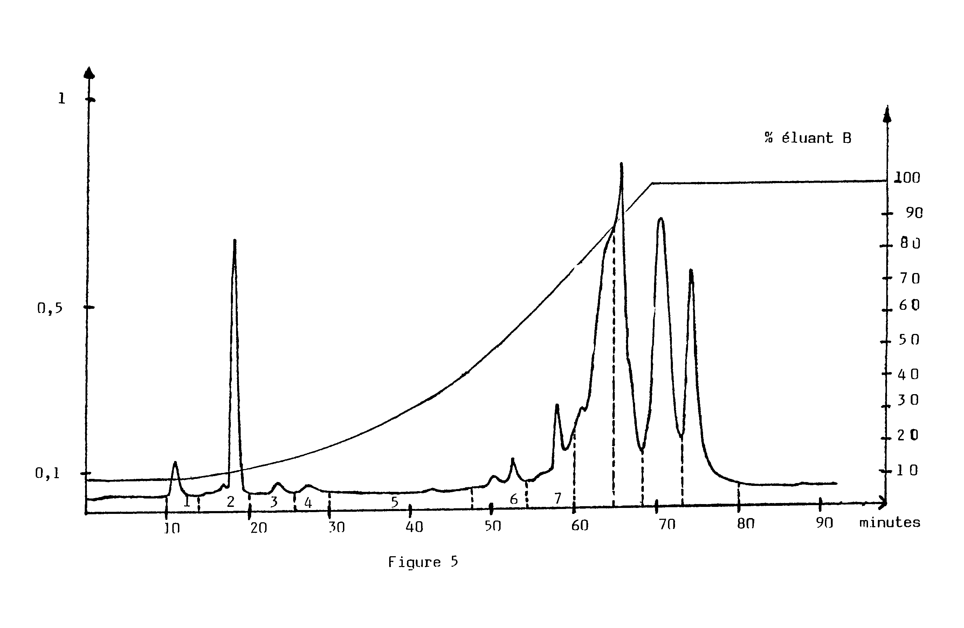

L'élution est effectuée en utilisant un gradient hyperbolique d'élution (gradient 7 de WATERS) et en opérant selon le tableau utilisé pour le MJH 228 ci-dessus. Le diagramme d'élution est représenté par la figure 5. Le diagramme d'élution contient 12 fractions. Les fractions éluées entre 60 et 65 minutes, 65 et 68 minutes et 68 et 74 minutes contiennent respectivement les substances MJH 253, MJH 254 et MJH 255.

La fraction contenant le produit MJH 254 est à nouveau purifiée par HPLC sur une colonne de même nature que celle utilisée précédemment, en recueillant des fractions de 0,5 $cm^3$, la vitesse d'élution étant de 1 $cm^3$/minute.

On utilise les mêmes éluants que précédemment.

L'élution est effectuée en opérant selon le tableau suivant :

| Temps (minutes) | Eluant A | Eluant B | Gradient |
|---|---|---|---|
| 30 | 60 | 40 | isocratique |
| 70 | 0 | 100 | linéaire |

Le diagramme d'élution contient 3 zones principales.

Les fractions éluées entre 15 et 20 minutes, 20 et 24 minutes et 46 et 60 minutes contiennent respectivement les substances MJH 299, MJH 301 et MJH 305.

La fraction MJH 299 est à nouveau purifiée par HPLC sur une colonne de même nature que celle utilisée précédemment, en recueillant des fractions de 0,5 $cm^3$, la vitesse d'élution étant de 1 $cm^3$/minute.

On utilise les mêmes éluants que précédemment.

0169115

L'élution est effectuée en opérant selon le tableau suivant:

| Temps (minutes) | Eluant A | Eluant B | Gradient |
|---|---|---|---|
| 30 | 80 | 20 | isocratique |
| 75 | 60 | 40 | linéaire |

Les fractions éluées entre 52 et 53 minutes et entre 54 et 57 minutes contiennent respectivement les substances actives MJH 335 et MJH 336.

La présente invention concerne également les compositions utilisables en thérapeutique qui contiennent une substance selon la présente invention en association avec un ou plusieurs diluants ou adjuvants compatibles et pharmaceutiquement acceptables.

Ces compositions peuvent être utilisées comme adjuvants de vaccins (par exemple vaccin antigrippal composé de sous-unités hemagglutinantes, vaccin antipoliomyélitique à virus inactivé, vaccin antimalarique) en injection simultanée avec l'antigène (viral, bactérien, parasitaire, fongique, tumoral) à l'égard duquel on souhaite augmenter la production d'anticorps ou la réactivité cellulaire spécifique.

Ces compositions pharmaceutiques peuvent être également employées comme immunostimulants non spécifiques en vue d'augmenter la résistance de l'hôte (homme ou animal domestique) vis-à-vis des infections ou en immunothérapie antitumorale.

En tant qu'adjuvants, les produits peuvent être administrés soit en solution aqueuse, soit en émulsion huileuse, soit encore sous forme de liposomes avec l'antigène à l'égard duquel on souhaite obtenir une réponse immunitaire augmentée ou améliorée par la voie utilisée pour cet antigène et dans des proportions variant entre 0,01 et 10 fois la quantité d'antigène avec lequel ils sont mélangés avant d'être injectés.

Pour l'application comme immunostimulants non spécifiques, ces produits peuvent être utilisés par voie intraveineuse, intramusculaire, sous-cutanée, intra-nasale, éventuellement orale ou rectale, soit en solution aqueuse soit en émulsion huileuse, soit encore sous forme de liposomes. Dans ce cas, la dose de composé selon l'invention qui est administrée est généralement comprise entre 0,01 et 10 mg/kg . En thérapeutique humaine, les doses journalières dépendent de l'effet recherché. Elles peuvent être comprises entre 0,5 et 10 mg pour un adulte.

Les compositions solides pour administration orale peuvent être des comprimés, des pilules, des poudres ou des granulés.

Comme compositions liquides pour administration orale, on peut utiliser des émulsions pharmaceutiquement acceptables, des solutions, des suspensions, des sirops et des élixirs.

Les compositions pour administration parentérale ou intra-nasale peuvent être des solutions stériles aqueuses, des suspensions ou des émulsions.

La stérilisation peut se faire de plusieurs façons, par exemple à l'aide d'un filtre bactériologique ou en incorporant des agents stérilisants. Les compositions solides rendues stériles par irradiation (rayons β ) peuvent être dissoutes dans de l'eau stérile ou tout autre milieu stérile injectable éventuellement au moment de l'emploi.

Les compositions pour administration rectale sont des suppositoires.

L'exemple suivant, donné à titre non limitatif, illustre une composition selon l'invention.

Exemple :

On prépare selon la technique habituelle une composition liquide administrable par voie intraveineuse ayant la composition suivante :

- substance MJH 257........................ 10 mg
- soluté injectable........................ 5 cm3

REVENDICATIONS

1 - Procédé de préparation de nouvelles substances biologiquement actives caractérisé en ce que l'on traite du fibrinogène bovin préalablement réduit par le mercaptoéthanol et alkylé par l'iodo-acétamide, par au moins une enzyme protéolytique, puis fractionne les produits hydrosolubles en fonction de leur poids moléculaire moyen, par filtration sur un ou plusieurs supports appropriés et isole les substances actives.

2 - Procédé selon la revendication 1 caractérisé en ce que l'enzyme protéolytique est choisie parmi la trypsine, la chymotrypsine ou leurs mélanges.

3 - Procédé selon la revendication 1 caractérisé en ce que l'on traite par une enzyme protéolytique du fibrinogène bovin réduit et alkylé soluble dans l'eau, le rapport enzyme/substrat étant voisin de 1/100, pendant 24 heures à 37°C, puis, après centrifugation, fractionne les produits hydrosolubles par filtration sur Sephadex G 50 en éluant avec de l'acide acétique à 30% puis isole les substances actives après purification par chromatographie liquide à haute performance.

4 - Nouvelles substances biologiquement actives lorsqu'elles sont obtenues selon le procédé de l'une des revendications 1, 2 ou 3.

5 - Nouvelles substances selon la revendication 4 caractérisées en ce que leur poids moléculaire est compris entre 500 et 7500.

6 - Composition pharmaceutique caractérisée en ce qu'elle contient au moins une substance selon les revendications 4 ou 5 en association avec un ou plusieurs diluants ou adjuvants compatibles.

Figure 1

Figure 2

Figure 3

Figure 4

d.o.
280 nm

% de l'éluant B

Pl. IV/5

0169115

Figure 5

# RAPPORT DE RECHERCHE EUROPEENNE

**Office européen des brevets**

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| A | CHEMICAL ABSTRACTS, vol. 70, no. 21, 26 mai 1969, pae 13, no. 93189b, Columbus, Ohio, US; A.Z. BUDZYNSKI et al.: "Partial reduction of bovine fibrinogen by some sulfhydryl compounds" & BIOCHIM. BIOPHYS. ACTA 1969, 175(2), 282-9 * Résumé * | 1-6 | C 07 K 3/10 C 07 K 15/06 A 61 K 39/39 A 61 K 45/05 A 61 K 37/18 |
| A | CHEMICAL ABSTRACTS, vol. 98, no. 19, 9 mai 1983, pae 374, no. 158997p, Columbus, Ohio, US; S. AHLSTEDT et al.: "Effects of fibrinogen ragment and proteolytic enzyme on the immune system in mice" & INT. ARCH. ALLERGY APPL. IMMUNOL. 1983, 70(1), 92-5 * Résumé * | 1-6 | |
| A | EP-A-0 049 666 (I.N.S.E.R.M.) * Revendications; page 2, lignes 25-29 * | 1-6 | DOMAINES TECHNIQUES RECHERCHES (Int. Cl.4) A 61 K C 07 K |
| A | FR-A-2 382 237 (BAYER AG) * Revendications 1,3,4,6,7; page 8, lignes 25-31 * | 1-6 | |

Le présent rapport de recherche a été établi pour toutes les revendications

| Lieu de la recherche | Date d'achèvement de la recherche | Examinateur |
|---|---|---|
| LA HAYE | 01-10-1985 | RYCKEBOSCH A.O.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82